(19) Europäisches Patentamt
European Patent Office
Office européen des brevets

(11) **EP 3 108 907 A1**

(12) # EUROPEAN PATENT APPLICATION
published in accordance with Art. 153(4) EPC

(43) Date of publication:
**28.12.2016 Bulletin 2016/52**

(21) Application number: **15752439.8**

(22) Date of filing: **19.02.2015**

(51) Int Cl.:
***A61M 1/02*** *(2006.01)* ***B01D 63/02*** *(2006.01)*
***B01D 69/08*** *(2006.01)* ***B01D 71/68*** *(2006.01)*

(86) International application number:
**PCT/JP2015/054562**

(87) International publication number:
**WO 2015/125852 (27.08.2015 Gazette 2015/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.02.2014 JP 2014029496**

(71) Applicant: **Toray Industries, Inc.**
**Tokyo 103-8666 (JP)**

(72) Inventors:
- **NOSAKA, Shiro**
  **Otsu-shi**
  **Shiga 520-8558 (JP)**
- **UENO, Yoshiyuki**
  **Otsu-shi**
  **Shiga 520-8558 (JP)**
- **OSABE, Masahiro**
  **Otsu-shi**
  **Shiga 520-8558 (JP)**
- **KISHIKAWA, Tatsuya**
  **Otsu-shi**
  **Shiga 520-8558 (JP)**

(74) Representative: **Kador & Partner**
**Corneliusstraße 15**
**80469 München (DE)**

(54) **HOLLOW FIBER MEMBRANE MODULE FOR CLEANING PLATELET SUSPENSION**

(57) The present invention aims to provide a hollow fiber membrane module capable of producing washed platelets having a low total protein amount and a high total platelet count from a platelet suspension. The present invention provides a hollow fiber membrane module for washing platelets by removal of impurities from a platelet suspension, comprising: a housing having a platelet suspension inlet, washed platelet outlet, and filtrate outlet; and a hollow fiber membrane for filtering the platelet suspension, wherein pores through which the platelets do not pass, while the impurities pass, are formed, which hollow fiber membrane is arranged inside the housing; wherein the capacity of the inlet-side space which communicates with the platelet suspension inlet and stores the platelet suspension before being filtered through the hollow fiber membrane in the housing is 30 to 400 mL, and the module water permeability is 50 to 300 mL/Pa/hr.

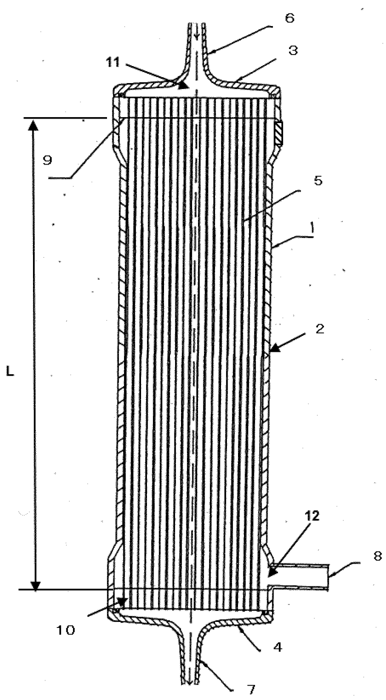


**Fig. 1**

EP 3 108 907 A1

**Description**

TECHNICAL FIELD

The present invention relates to a hollow fiber membrane module for removal of protein in a platelet suspension by washing.

BACKGROUND ART

In production of platelet preparations, blood components collected from blood donors are centrifuged to remove blood cell components from the blood, and the preparations are provided as platelet suspensions in which platelets are suspended in blood plasma. In ordinary platelet preparations, impurities such as proteins are remaining in the plasma. Therefore, in transfusion of a platelet preparation, the proteins in the plasma may act as a cause of nonhemolytic blood transfusion reaction. For reducing the frequency of occurrence of such nonhemolytic blood transfusion reaction, it has been recommended to use platelets washed by removing impurities such as proteins (washed platelets). Washed platelets are produced by physically separating/removing impurities such as proteins from a platelet suspension. Examples of methods for separating and removing proteins from a platelet suspension include centrifugation method and membrane filtration method.

Conventionally, centrifugation method is carried out for production of washed platelets. In the centrifugation method, a platelet suspension as a material is centrifuged, and the resulting supernatant, which contains proteins, is removed, followed by adding a preservation solution to the concentrated platelets. On the other hand, in the membrane filtration method, proteins are removed by filtration of a platelet suspension. For example, a plasma separation membrane module to be used for membrane filtration based on extracorporeal circulation has been reported (Patent Document 1). Specific examples of the membrane filtration reported so far include a method in which proteins are removed from a platelet suspension by cross-flow filtration, and a method in which a platelet suspension is subjected to dead-end filtration through a membrane (Patent Documents 2 and 3).

PRIOR ART DOCUMENTS

[Patent Documents]

[Patent Document 1] JP 1-171566 A
[Patent Document 2] JP 2012-143554 A
[Patent Document 3] JP 2012-176081 A

SUMMARY OF THE INVENTION

PROBLEMS TO BE SOLVED BY THE INVENTION

However, centrifugation method, which has been conventionally used for production of washed platelets, causes serious damage to platelets, and there are also problems such as activation, and generation of aggregates. Moreover, since complete removal of the supernatant is difficult, a plurality of times of separation needs to be carried out for sufficient reduction of the total protein amount. Therefore, there are problems such as a laborious operation, a long processing time and a low platelet recovery rate.

In the membrane filtration methods by cross-flow filtration described in Patent Documents 1 and 2, proteins are removed by cross-flow filtration in which a flow parallel to a membrane and a flow filtered through the membrane are allowed to flow at arbitrary ratios. Therefore, proteins are not removed from the flow parallel to the membrane. Thus, a plurality of times of separation needs to be carried out for sufficient reduction of the total protein amount. Therefore, there are problems such as a laborious operation, a long processing time, increased platelet activation and a low platelet recovery rate.

In the method described in Patent Document 3, in which a platelet suspension is subjected to dead-end filtration through a membrane, the protein removal rate is high, but clogging of the membrane with platelets is likely to occur, and the platelets clogging the membrane are not recovered, resulting in a low platelet recovery rate, which is problematic. Although the document describes detachment of blood components clogging the membrane, the filtration rate of the membrane decreased to not more than 20%. Therefore, the effect of suppression of clogging was insufficient. The document does not mention the recovery rate.

Thus, although a high protein removal rate and a high platelet recovery rate are necessary for washing platelets by removing impurities such as proteins from a platelet suspension to produce washed platelets, there has not conventionally been a module for production of washed platelets showing both a high protein removal rate and a high platelet recovery rate.

An object of the present invention is to provide a hollow fiber membrane module for washing of a platelet suspension, which module is capable of achieving both a high protein removal rate and a high platelet recovery rate by suppressing clogging of the hollow fiber membrane with platelets.

MEANS FOR SOLVING THE PROBLEMS

In order to solve the problems described above, the present inventors intensively studied to discover the following inventions (1) to (9).

(1) A hollow fiber membrane module for washing platelets by removal of impurities from a platelet suspension, comprising:

a housing having a platelet suspension inlet, washed platelet outlet, and filtrate outlet; and
a hollow fiber membrane for filtering the platelet suspension, wherein pores through which the platelets do not pass, while the impurities pass, are formed, the hollow fiber membrane being arranged inside the housing;

wherein
the capacity of the inlet-side space which communicates with the platelet suspension inlet and stores the platelet suspension before being filtered through the hollow fiber membrane in the housing is 30 to 400 mL, and the module water permeability is 50 to 300 mL/Pa/hr.
(2) The hollow fiber membrane module according to (1), wherein the ratio (L/A) of the effective length (L) of the hollow fiber membrane to the cross-sectional area (A) of the inlet-side space vertical to the longitudinal direction of the housing is 250 to 1300 $m^{-1}$.
(3) The hollow fiber membrane module according to (1) or (2), wherein the maximum pressure of filtration pressure during dead-end filtration of 200 mL of a platelet suspension containing $1.25 \times 10^9$ platelets/mL at a flow rate of 50 mL/min is not more than 30 kPa.
(4) The hollow fiber membrane module according to any one of (1) to (3), wherein the ratio of pore areas on the surface of the hollow fiber membrane facing the inlet-side space is 10 to 30%.
(5) The hollow fiber membrane module according to any one of (1) to (4), wherein the hollow fiber membrane is a membrane composed of a polysulfone-based polymer.
(6) The hollow fiber membrane module according to any one of (1) to (5), wherein the abundance ratio of hydrophilic polylmers to the total molecules from the surface of the hollow fiber membrane facing the inlet-side space to a depth of 10 nm is 40 to 60% by mass.
(7) The hollow fiber membrane module according to any one of (1) to (6), wherein the abundance ratio of carbon atoms derived from ester groups to the total carbon atoms from the surface of the hollow fiber membrane facing the inlet-side space to a depth of 10 nm is 0.1 to 10 atomic percent.
(8) A platelet suspension washing device comprising:

the hollow fiber membrane module according to any one of (1) to (7); and
an air chamber which is arranged upstream of the platelet suspension inlet, and has a capacity of 1 to 30 mL.

(9) The platelet suspension washing device according to (8), wherein a roller pump is arranged upstream of the air chamber along the liquid current of the platelet suspension.

EFFECT OF THE INVENTION

According to the present invention, by use of a hollow fiber membrane module having both an increased protein removal rate and an increased platelet recovery rate, it is possible to produce washed platelets having a low total protein amount and a high total platelet count.

BRIEF DESCRIPTION OF THE DRAWINGS

Fig. 1 is a longitudinal cross-sectional view of a hollow fiber membrane module for the internal pressure method according to a first embodiment of the present invention.

Fig. 2 is a cross-sectional view of a hollow fiber membrane module for the internal pressure method according to the first embodiment of the present invention, which cross section is vertical to the longitudinal direction of the module.

Fig. 3 is a longitudinal cross-sectional view of a hollow fiber membrane module for the external pressure method according to a second embodiment of the present invention.

Fig. 4 is a cross-sectional view of a hollow fiber membrane module for the external pressure method according to the second embodiment of the present invention, which cross section is vertical to the longitudinal direction of the module.

Fig. 5 is a schematic view of a platelet suspension washing device using the hollow fiber membrane module for the internal pressure method according to the first embodiment of the present invention.

MODE FOR CARRYING OUT THE INVENTION

The hollow fiber membrane module of the present invention is a hollow fiber membrane module for washing platelets by removal of impurities from a platelet suspension. The hollow fiber membrane module is characterized in that it comprises a housing having a platelet suspension inlet, washed platelet outlet, and filtrate outlet; and a hollow fiber membrane for filtering the platelet suspension, wherein pores through which the platelets do not pass, while the impurities pass, are formed, which hollow fiber membrane is arranged inside the housing; wherein the capacity of the inlet-side space which communicates with the platelet suspension inlet and stores the platelet suspension before being filtered through the hollow fiber membrane in the housing is 30 to 400 mL, and the module water permeability is 50 to 300 mL/Pa/hr.

Preferred embodiments of the present invention are described below in detail with reference to drawings. However, the present invention is not limited to these embodiments. The ratios in the drawings are not necessarily the same as those in the description.

As a first embodiment according to the present invention, a hollow fiber membrane module for the internal pressure method is shown in Fig. 1 and Fig. 2. Fig. 1 is a longitudinal cross-sectional view of the hollow fiber membrane module for the internal pressure method 1. Fig. 2 is a cross-sectional view of the hollow fiber membrane module for the internal pressure method 1, which cross section is vertical to the longitudinal direction of the module. In cases of a hollow fiber membrane module for the internal pressure method, filtration is carried out by allowing a platelet suspension to flow through the hollow portion of each hollow fiber membrane.

The hollow fiber membrane module for the internal pressure method 1 has a constitution composed of a cylindrical member 2; a housing having headers 3 and 4 that are fluid-tightly connected and immobilized at both ends of the cylindrical member 2; and a bundle of hollow fiber membranes 5 stored in the housing. At the top portion of the header 3, a platelet suspension inlet 6 for introducing a platelet suspension into the hollow fiber membrane module, having a protruding shape, is formed. At the top portion of the header 4, a washed platelet outlet 7 having a protruding shape for releasing a liquid containing washed platelets separated from the platelet suspension by filtration through the bundle of the hollow fiber membranes 5 is formed. On the lateral part in the header 4 side of the cylindrical member 2, a filtrate outlet 8 for discharging a filtrate containing impurities such as proteins separated from the platelet suspension is formed.

The bundle of the hollow fiber membranes 5 is arranged along the entire length in the longitudinal direction in the cylindrical member 2, and both ends of the hollow fiber membranes 5 are immobilized in the cylindrical member 2 by a partition wall 9 in the header 3 side and a partition wall 10 in the header 4 side formed with a cured potting material such that the openings of hollow fiber membrane hollow portions 13 as the lumens of the hollow fiber membranes 5 are not closed.

After introduction of a platelet suspension from the platelet suspension inlet 6, the platelet suspension flows into an inlet-side space 11. The inlet-side space 11 herein means the space through which the platelet suspension before the filtration flows. In the hollow fiber membrane module for the internal pressure method 1, the inlet-side space 11 means the space including: the space surrounded by the header 3 and the partition wall 9, and the space surrounded by the header 4 and the partition wall 10, shown in Fig. 1; and the spaces in the hollow fiber membrane hollow portions 13, shown in Fig. 2. The inlet-side space 11 communicates with the platelet suspension inlet 6 and the washed platelet outlet 7. The inlet-side space can also be called a platelet-side space.

The platelet suspension that flows in the inlet-side space 11 is filtered by being passed through pores present on the surface of the hollow fiber membranes 5, and the filtrate containing impurities such as proteins passes the pores into a filtrate-side space 12. The filtrate-side space 12 means the space into which the filtrate containing impurities such as proteins flows after passing the pores of the hollow fiber membranes. In the hollow fiber membrane module for the internal pressure method 1, the filtrate-side space 12 means the space surrounded by the cylindrical member 2 and the partition walls 9 and 10, excluding the hollow fiber membranes 5 and the hollow fiber membrane hollow portions 13. The

filtrate-side space 12 communicates with the filtrate outlet 8.

As a second embodiment according to the present invention, a hollow fiber membrane module for the external pressure method is shown in Fig. 3 and 4. Fig. 3 is a longitudinal cross-sectional view of a hollow fiber membrane module for the external pressure method 14. Fig. 4 is a cross-sectional view of the hollow fiber membrane module for the external pressure method 14, which cross section is vertical to the longitudinal direction of the module. In cases of a hollow fiber membrane module for the external pressure method, filtration is carried out by allowing a platelet suspension to flow through the space outside the hollow fiber membranes. For the hollow fiber membrane module for the external pressure method as the second embodiment, the same numbers are given to members having the same functions.

The hollow fiber membrane module for the external pressure method 14 has a constitution composed of a cylindrical member 2; a housing having headers 3 and 4 that are fluid-tightly connected and immobilized at both ends of the cylindrical member 2; and a bundle of hollow fiber membranes 5 stored in the housing. On the lateral part in the header 3 side of the cylindrical member 2, a platelet suspension inlet 6 for introducing a platelet suspension into the hollow fiber membrane module is formed. On the lateral part in the header 4 side of the cylindrical member 2, washed platelets outlet 7 having a protruding shape for releasing a liquid containing washed platelets separated from the platelet suspension by filtration through the bundle of the hollow fiber membranes 5 is formed. At the top portion of the header 4, a filtrate outlet 8 for discharging a filtrate containing unnecessary proteins separated from the platelet suspension is formed.

The bundle of the hollow fiber membranes 5 is arranged along the entire length in the longitudinal direction of the cylindrical member 2, and both ends of the hollow fiber membranes 5 are immobilized in the cylindrical member 2 by a partition wall 9 in the header 3 side and a partition wall 10 in the header 4 side formed with a cured potting material such that the openings of the hollow fiber membrane hollow portions 13 as the lumens of the hollow fiber membranes 5 are not closed.

In the first embodiment, the ends of the hollow fiber membranes 5 in the side more distant from the filtrate outlet 8 are open. In contrast, in the hollow fiber membrane module for the external pressure method, the ends of the hollow fiber membranes 5 in the side more distant from the filtrate outlet 8 may be closed, or may be folded into the U-shape.

After introduction of a platelet suspension from the platelet suspension inlet 6, the platelet suspension flows into an inlet-side space 11. The inlet-side space 11 herein means the space in which the platelet suspension before the filtration is retained. In the hollow fiber membrane module for the external pressure method 14, the inlet-side space 11 means the space surrounded by the cylindrical member 2 and the partition walls 9 and 10, excluding the hollow fiber membranes 5 and the hollow fiber membrane hollow portions 13. The inlet-side space 11 communicates with the platelet suspension inlet 6 and the washed platelet outlet 7.

In the hollow fiber membrane module for the external pressure method 14, the filtrate-side space 12 means the space including: the space surrounded by the header 3 and the partition wall 9, and the space surrounded by the header 4 and the partition wall 10; and the spaces in the hollow fiber membrane hollow portions 13. The filtrate-side space 12 communicates with the filtrate outlet 8.

A platelet suspension washing device using a hollow fiber membrane module of the present invention is shown using Fig. 5. Fig. 5 is a schematic view of a platelet suspension washing device using the hollow fiber membrane module for the internal pressure method according to the first embodiment of the present invention. A bag for storing a platelet suspension and a bag for storing a preservation solution are arranged in parallel in the most upstream of a circuit connected to the platelet suspension inlet 6, and tube clamps 17 are arranged downstream thereof such that the connection to the circuit can be switched. Between the platelet suspension/preservation solution and the hollow fiber membrane module for the internal pressure method 1, a pump 16 for feeding the platelet suspension and the preservation solution, and an air chamber 15 for preventing inclusion of gas into the hollow fiber membrane module for the internal pressure method 1, are arranged. A bag for storing the filtrate is arranged downstream of a circuit connected to the filtrate outlet 8, and a bag for storing washed platelets is arranged downstream of a circuit connected to the washed platelet outlet 7. A tube clamp 17 is arranged upstream of each bag such that the connection to the circuit can be switched.

Here, the platelet suspension means a liquid prepared by removing blood cell components from blood and separating/collecting platelets and plasma. The platelet suspension may contain an anticoagulant such as citric acid, and/or a preservation solution.

Here, the preservation solution means a liquid for stably suspending platelets therein. As the preservation solution, a liquid containing bicarbonate is preferably used.

Here, the washing liquid means a liquid for stably washing platelets. Similarly to the preservation solution, as the washing liquid, a liquid containing bicarbonate is preferably used for stable washing.

The method for producing washed platelets from a platelet suspension using the hollow fiber membrane module of the present invention is not limited, and specific examples of the method for producing washed platelets include the following.

A method for producing washed platelets from a platelet suspension comprises: a filtration step of subjecting a platelet suspension to dead-end filtration from the inlet-side space to the filtrate-side space of the hollow fiber membrane

module, to allow a filtrate containing impurities such as proteins to pass into the filtrate-side space, thereby separating a liquid containing platelets from the filtrate containing impurities such as proteins; a washing step of allowing a washing liquid to flow from the inlet-side space to the filtrate-side space, to allow impurities such as proteins remaining in the liquid containing platelets to pass into the filtrate-side space, thereby removing the impurities such as proteins; and a recovering step of recovering a liquid containing washed platelets by allowing a preservation solution to flow through the inlet-side space.

In the filtration step, the platelet suspension is introduced from the platelet suspension inlet of the hollow fiber membrane module, and dead-end filtration is carried out from the inlet-side space toward the filtrate-side space. The filtrate containing impurities such as proteins in the platelet suspension passes the hollow fiber membranes, and flows into the filtrate-side space, followed by being discharged from the communicating filtrate outlet. On the other hand, platelets in the platelet suspension cannot pass the hollow fiber membranes, and remain in the inlet-side space.

In the washing step, the washing liquid is similarly subjected to dead-end filtration, wherein impurities such as proteins remaining in the inlet-side space pass the hollow fiber membranes, and flow into the filtrate-side space, followed by being discharged from the communicating filtrate outlet as a filtrate containing impurities such as proteins.

Thereafter, in the recovering step, the preservation solution is introduced from the platelet suspension inlet into the inlet-side space to mix the platelets with the preservation solution, and the resulting mixture is released from the washed platelet outlet as a liquid containing platelets. In this process, in cases where the volume of the platelet suspension used as the material is the same as the volume of the preservation solution, washed platelets having the same platelet concentration as that before the treatment can be obtained.

Since, by reducing the capacity of the inlet-side space, the volume of the liquid filtered in the filtration step can be increased, the amount of impurities such as proteins remaining in the inlet-side space can be reduced. Moreover, since the volume of the platelet suspension in the inlet-side space can be decreased in the washing step, the washing efficiency can be increased, and the removal rate of impurities such as proteins can be increased even with a small volume of the washing liquid. In the recovering step, since the volume of the preservation solution used for the recovery relative to the liquid volume in the inlet-side space decreases, the recovery rate of platelets decreases. On the other hand, in cases where the capacity of the inlet-side space is too small, the concentration of platelets remaining in the inlet-side space is high in the filtration step, so that activation and aggregation of platelets are more likely to occur due to interactions between platelets. Thus, the capacity of the inlet-side space of the hollow fiber membrane module needs to be not less than 30 mL, and is preferably not less than 70 mL. The capacity needs to be not more than 400 mL, and is preferably not more than 200 mL.

In conventional hollow fiber membrane modules, during filtration of a platelet suspension by dead-end filtration, the filtration pressure increases due to clogging of the surfaces of the hollow fiber membranes with platelets. Due to the increase in the filtration pressure, the platelets are strongly pushed against the membranes, and this causes aggregation of the platelets, leading to further progress of the clogging. When aggregation of the platelets occurs, the platelets adhere to the surfaces of the hollow fiber membranes. In such cases, the platelets cannot be detached by the flow of the preservation solution for recovery of platelets, and remain on the surfaces of the hollow fiber membranes, resulting in a decrease in the platelet recovery rate. Moreover, in cases where filtration is carried out by the internal pressure method, in which a platelet suspension is allowed to flow through the hollow fiber membrane hollow portions, aggregated platelets cause occlusion of the hollow portions. In these cases, the platelet recovery rate is low since, even if the preservation solution is allowed to flow for recovery of platelets, the preservation solution cannot easily pass through hollow fiber membrane hollow portions. In particular, since platelet suspensions used for blood transfusion often have 3-fold or higher concentrations of platelets than the platelet concentration in the body, they are more likely to cause clogging during the filtration.

In order to suppress clogging and to increase the platelet recovery rate in filtration of a platelet suspension by dead-end filtration, it is necessary to maintain a state where the filtration pressure is low, and platelet aggregation is suppressed. In cases where the water permeability of the hollow fiber membrane module is low, the filtration pressure is high, so that the platelet recovery rate is low.

The water permeability of a hollow fiber membrane module depends on the water permeability and the membrane area of the hollow fiber membranes contained therein. Therefore, an increase in the water permeability is accompanied by an increase(s) in the membrane area and/or the water permeability of the hollow fiber membranes. However, in cases where the membrane area of the hollow fiber membranes is increased, the size of the hollow fiber membrane module increases, causing problems such as difficulty in handling and an increase in the volume of the liquid for washing before the use of the hollow fiber membrane module. In general, for the purpose of increasing the water permeability of a hollow fiber membrane, the pore size of the hollow fiber membrane is increased. However, in cases where the pore size is increased, platelets are more likely to penetrate the membrane.

In view of this, the present inventors carried out experiments, and, as a result, found optimal water permeability of the hollow fiber membrane with which the platelet recovery rate can be increased while ease of handling of the hollow fiber membrane module is maintained. More specifically, the water permeability of the hollow fiber membrane needs to

be not less than 2.5 mL/Pa/hr, and is preferably not less than 4 mL/Pa/hr. The water permeability of the hollow fiber membrane needs to be not more than 15 mL/Pa/hr, and is preferably not more than 13 mL/Pa/hr.

As a result of experiments, it was found that the ratio of pore areas on the surfaces of the hollow fiber membranes in the inlet-side space is preferably increased for suppression of the increase in the filtration pressure due to clogging. More specifically, the ratio of pore areas on the surfaces of the hollow fiber membranes in the inlet-side space is preferably not less than 10%, more preferably not less than 12%. On the other hand, in cases where the ratio of pore areas is too high, the strength of the membrane is insufficient, so that the ratio of pore areas is preferably not more than 30%, more preferably not more than 20%. The ratio of pore areas means the ratio of the total area of the pores on the surfaces of the hollow fiber membranes to the area of the surfaces of the hollow fiber membranes. The method for measuring the ratio of pore areas is described later in detail. The ratio of pore areas can be obtained by subjecting an image taken with an electron microscope at a magnification of x1000 to image processing using known software such as atrox Inspector 2.2 (Matrox Electronic Systems Ltd.).

From the viewpoint of increasing the platelet recovery rate while suppressing the platelet aggregation, the filtration pressure in the filtration step is preferably not more than 30 kPa. As the material of washed platelets, 5, 10, 15, or 20 units of a platelet suspension is commonly used. In particular, 10 units of a platelet suspension is most frequently used. In this standard, 10 units of a platelet suspension means that the platelet concentration is $8.3 \times 10^8$ platelets/mL to $1.8\times10^9$ platelets/mL, and that the liquid volume is 160 mL to 240 mL. In the hollow fiber membrane module of the present invention, the maximum pressure of filtration pressure during dead-end filtration of 200 mL of a platelet suspension containing $1.25\times10^9$ platelets/mL, which is 10 units of a platelet preparation, at a flow rate of 50 mL/min is preferably not more than 30 kPa, more preferably not more than 20 kPa.

In the filtration step, a shear stress is applied to the platelets due to contact of the platelets with the surfaces of the hollow fiber membranes in the feeding space side. It is known that application of a high shear stress to platelets causes activation of the platelets. Therefore, by reducing the shear stress applied to the platelets in the filtration step, aggregation of the platelets can be suppressed, and the platelet recovery rate can be increased. On the other hand, in the recovering step, the shear stress applied to the platelets is increased to allow easier detachment of platelets adhered to the surfaces of the hollow fiber membranes. This increases the platelet recovery rate. The shear stress applied to the platelets is proportional to the linear velocity of the flow in the hollow fiber membrane module.

As the ratio (L/A) of the effective length (L) of the hollow fiber membrane to the cross-sectional area (A) of the inlet-side space vertical to the longitudinal direction of the housing increases, the linear velocity increases. As L/A decreases, the linear velocity decreases. For example, in cases where the flow rate is within the range of 50 mL/min. to 500 mL/min., L/A is preferably not less than 250 $m^{-1}$, more preferably not less than 500 $m^{-1}$, from the viewpoint of increasing the linear velocity for detaching platelets adhered to the hollow fiber membranes to increase the platelet recovery rate. On the other hand, L/A is preferably not more than 1300 $m^{-1}$, more preferably not more than 700$m^{-1}$, from the viewpoint of decreasing the linear velocity for suppressing platelet aggregation to increase the platelet recovery rate.

The effective length of a hollow fiber membrane means the length of the hollow fiber membrane in which filtration is substantially possible, and corresponds to the length of the hollow fiber membrane excluding the partition walls and the portions embedded in the partition walls. The membrane area of the hollow fiber membrane module is calculated using this effective length as a standard.

In a hollow fiber membrane module for the internal pressure method, the cross-sectional area (A) of the inlet-side space vertical to the longitudinal direction of the housing is the cross-sectional area of the hollow fiber membrane hollow portions, and calculated according to Equation 1.

$$A = (ID / 2)^2 \times \pi \times n \text{ ... Equation 1}$$

A: Cross-sectional area of the inlet-side space vertical to the longitudinal direction of the housing ($m^2$)
ID: Hollow fiber inner diameter (m)
$\pi$: Circumference ratio
n: Number of hollow fiber membranes

Here, in cases where the hollow fiber membranes contained in the hollow fiber membrane module are composed of two or more kinds of hollow fiber membranes having different hollow fiber inner diameters, Equation 1 is applied to each kind of hollow fiber membranes, and the obtained values are integrated to calculate the cross-sectional area (A) of the inlet-side space vertical to the longitudinal direction of the housing.

In a hollow fiber membrane module for the external pressure method, the cross-sectional area (A) of the inlet-side space vertical to the longitudinal direction of the housing is a value obtained by subtracting the cross-sectional area of the hollow fiber membranes from the cross-sectional area of the housing at a position where the platelet suspension

inlet and the washed platelet outlet are absent, and calculated according to Equation 2 and Equation 3.

$$A_M = (OD / 2)^2 \times \pi \times n \text{ ... Equation 2}$$

$$A = A_H - A_M \text{ ... Equation 3}$$

A: Cross-sectional area of the inlet-side space vertical to the longitudinal direction of the housing ($m^2$)
$A_H$: Cross-sectional area of the housing
$A_M$: Cross-sectional area of the hollow fiber membranes
OD: Hollow fiber outer diameter (m)
$\pi$: Circumference ratio
n: Number of hollow fiber membranes

Here, in cases where the hollow fiber membranes contained in the hollow fiber membrane module are composed of two or more kinds of hollow fiber membranes having different hollow fiber outer diameters, Equation 2 is applied to each kind of hollow fiber membranes, and the obtained values are integrated to calculate the cross-sectional area of the hollow fiber membranes ($A_M$). The cross-sectional area of the housing is the mean calculated for the same section as that for the effective length of the hollow fiber membrane in the longitudinal direction of the housing. For example, in cases where the housing has a shape which continuously changes in the longitudinal direction, the cross-sectional area is measured at each of a total of five points positioned at the same intervals from one end to the other end of the section which is the same as that for the effective length of the hollow fiber membrane in the longitudinal direction, and the arithmetic mean of the measured values is calculated. In cases where the housing has a shape which discontinuously changes in the longitudinal direction, the cross-sectional area is measured in each of portions having different shapes, and each measured value is multiplied by the ratio of the corresponding portion in the section which is the same as that for the effective length of the hollow fiber membrane in the longitudinal direction, followed by calculating the sum of the obtained values to determine the mean of the cross-sectional area of the housing.

In the present invention, the hollow fiber membranes used for the hollow fiber membrane module are not limited as long as they are hollow fiber membranes produced using a material which suppresses platelet activation, that is, a material having blood compatibility. Hollow fiber membranes used in known methods for producing washed platelets by membrane filtration, for example, the hollow fiber membranes described in Patent Documents 2 and 3, may be preferably used.

Specific examples of the material of the hollow fiber membranes include, but are not limited to, polysulfone-based polymers, polystyrene, polyurethane, polyethylene, polypropylene, polycarbonate, polyvinylidene fluoride, and polyacrylonitrile. In particular, hollow fiber membranes produced using, as a main material, the so-called polysulfone-based polymer such as polysulfone or polyethersulfone are known to have excellent water permeability and fractionation performance. In the present invention, a polysulfone-based polymer is preferably used as the material. The polysulfone-based polymer means a polymer having an aromatic ring, sulfonyl group, and ether group in its backbone.

Examples of the polysulfone-based polymer include polysulfones represented by General Formula (I), polysulfones represented by General Formula (II), polyethersulfones, and polyallylethersulfones. Among these, polysulfones represented by General Formula (I), and polysulfones represented by General Formula (II), are preferred. The number n is more preferably 50 to 80. A block copolymer of a polysulfone represented by General Formula (I) or (II) and other monomers, or a modified body of a polysulfone represented by General Formula (I) or (II), may be used. The ratio of the polysulfone-derived structure in the block copolymer of a polysulfone represented by General Formula (I) or (II) and other monomers is preferably not less than 90% by mass with respect to the entire block copolymer.

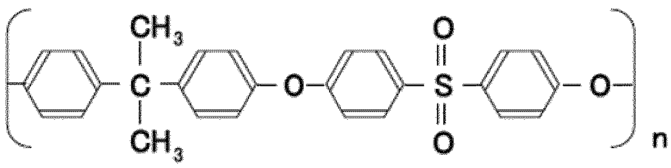 · · · (I)

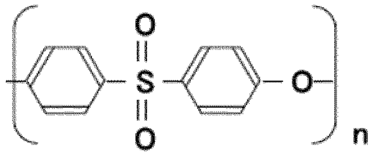 · · · (I I)

The inner diameter and the membrane thickness of the hollow fiber membranes are not limited. Hollow fiber membranes having an inner diameter of about 100 to 500 μm and a membrane thickness of about 30 to 200 μm may be preferably used.

The average pore size of the pores of the hollow fiber membranes is not limited as long as platelets do not pass through the pores, while impurities pass through the pores. Since the sizes of the platelets, especially human platelets, to be subjected to the washing treatment are 2 to 4 μm, the average pore size is not more than 1.5 μm, preferably not more than 1 μm.

Each hollow fiber membrane constituting the hollow fiber membrane bundle preferably contains, in order to prevent activation of platelets in contact with the hollow fiber membrane, a hydrophilic component at least on the surface which contacts platelets (for example, in cases of filtration by the internal pressure method, at least on the lumen-side surface of the hollow fiber membrane). The "hydrophilic component" herein means a substance which is easily soluble in water, having a solubility of not less than 10 g/100 g in pure water at 20°C. A hydrophilic polymer is preferably used as the hydrophilic component.

By the inclusion of the hydrophilic polymer on the surface of the hollow fiber membrane, the blood compatibility can be increased, and the platelet aggregation can be suppressed. From the viewpoint of suppression of the platelet aggregation, the abundance ratio of hydrophilic polymers to the total molecules in the portion from the surface of the hollow fiber membrane in the inlet-side space to a depth of 10 nm is preferably not less than 40% by mass. On the other hand, in cases where the hydrophilic polymer is present in an excess amount, elution of the hydrophilic polymer from the hollow fiber membrane may occur to cause contamination of the washed blood product. Moreover, swelling of the hydrophilic polymer on the surface of the hollow fiber membrane may cause narrowing of the pores of the hollow fiber membrane, leading to a decrease in the water permeability. Thus, the abundance ratio of hydrophilic polymers to the total molecules in the portion from the surface of the hollow fiber membrane in the inlet-side space to a depth of 10 nm is preferably not more than 60% by mass.

The hydrophilic polymer herein means a water-soluble polymer, or a water-insoluble polymer that interacts with water molecules by electrostatic interaction and/or hydrogen bonds. The hydrophilic polymer herein means a polymer that can be dissolved at a ratio of not less than 1000 ppm in pure water at 25°C. Specific examples of the hydrophilic polymer include, but are not limited to, polyalkylene glycols such as polyethylene glycol and polypropylene glycol; nonionic hydrophilic polymers such as polyvinyl alcohol, polyvinyl pyrrolidone, polyvinyl acetate, polyvinyl caprolactam, hydroxyethyl methacrylate, and methyl methacrylate; and ionic hydrophilic polymers such dextran sulfate, polyacrylic acid, polyethylenimine, and polyallylamine.

Examples of the method for including the hydrophilic polymer on the hollow fiber membrane surface include: coating by physical adsorption; thermal or radiation cross-linking; and chemical bonding by chemical reaction. In the process of producing a hollow fiber membrane, a membrane-forming liquid is discharged from a double annular nozzle while an injection liquid is allowed to flow inside. The hydrophilic polymer may be added to the injection liquid. In such a case, the hydrophilic polymer in the injection liquid is diffused into the membrane-forming liquid side before the phase separation of the hollow fiber membrane occurs to establish the membrane structure. Therefore, the hydrophilic polymer can be localized on the hollow fiber membrane surface.

The abundance ratio of hydrophilic polymers to the total molecules in the portion from the surface of the hollow fiber membrane in the inlet-side space to a depth of 10 nm can be calculated by carrying out measurement by X-ray electron spectroscopy (hereinafter referred to as "ESCA") at a measurement angle of 90° and investigating the abundance ratios of elements in the portion from the surface of the hollow fiber membrane to a depth of 10 nm. More specifically, the abundance ratio of hydrophilic polymers to the total molecules can be measured and calculated by the following method.

In cases of a hollow fiber membrane module for the internal pressure method, when the measurement is carried out for the portion from the surface of the hollow fiber membrane in the inlet-side space to a depth of 10 nm, the inner surface of the hollow fiber membrane is exposed by cutting the membrane into a semi-cylindrical shape using a single-edged blade. After rinsing the hollow fiber membrane with ultrapure water, the membrane is dried at room temperature at 0.5 Torr for 10 hours to provide a measurement sample. The sample is set in the apparatus, and the angle of the detector with respect to the angle of incidence of X-ray is adjusted such that the measurement angle becomes 90°. From the integrated intensity of the spectrum of each of CIs, N1s, and S2p, and the relative sensitivity coefficient specific to the apparatus, the abundance ratios of carbon atoms, nitrogen atoms, and sulfur atoms are determined.

Here, for example, in cases where polysulfone and a hydrophilic polymer material polyvinyl pyrrolidone are used as materials of the hollow fiber membrane, the abundance ratio of polyvinyl pyrrolidone on the surface is calculated according to the following Equation 4.

$$\text{Abundance ratio of polyvinyl pyrrolidone on the surface (\% by mass)} = N \times 111 / (N \times 111 + S \times 442) \text{ ... Equation 4}$$

N: Abundance ratio of nitrogen atoms
S: Abundance ratio of sulfur atoms
111: Number of repeat units of polyvinyl pyrrolidone
442: Number of repeat units of polysulfone-based polymer

As mechanisms of adhesion of platelets to the hollow fiber membrane surface, there are two pathways. In the first pathway, activation of platelets immediately occurs when the platelets contact the hollow fiber membrane surface, and this activation causes aggregation and adhesion of the platelets. In the second pathway, proteins involved in blood coagulation such as fibrinogen adhere to the surface of the hollow fiber membrane, and activate platelets to induce adhesion of the platelets. Thus, for suppression of the adhesion of platelets to the hollow fiber membrane surface, it is necessary to prevent approach of platelets to the hollow fiber membrane surface, and adhesion of proteins such as fibrinogen to the hollow fiber membrane surface.

As means for preventing approach of platelets to the hollow fiber membrane surface, formation of a diffuse layer with a hydrophilic polymer on the hollow fiber membrane surface is effective. The excluded volume effect by the diffuse layer prevents platelets from approaching the hollow fiber membrane surface. By the formation of the diffuse layer, adhesion of proteins such as fibrinogen to the hollow fiber membrane surface can also be prevented. However, in cases where the hydrophilicity of the diffuse layer is too high, bound water in the vicinity of proteins is trapped in the diffuse layer, and this causes structural changes of the proteins, resulting in adhesion of the proteins to the hollow fiber membrane surface. Thus, the effect to suppress adhesion of proteins such as fibrinogen decreases. The bound water herein means water which is present in the vicinity of proteins and whose movement is restricted by hydrogen bonds. It is thought that bound water stabilizes the structures of proteins.

Examples of hydrophilic polymers preferred for formation of the diffuse layer include water-insoluble polymers having a rather hydrophobic unit, such as vinyl caprolactam, propylene glycol, vinyl acetate, hydroxyethyl methacrylate, and methyl methacrylate. Polymers having an ester group are more preferred. Polymers having a side-chain type ester group such as a vinyl acetate group or methyl acrylate group are still more preferred. It is assumed that side-chain type ester groups such as a vinyl acetate group and methyl acrylate group do not trap bound water since they are moderately hydrophilic. On the other hand, highly hydrophobic polymers such as polyethylene terephthalate are not preferred even in cases where they have an ester group.

Since homopolymers of units such as vinyl caprolactam, propylene glycol, vinyl acetate, hydroxyethyl methacrylate, and methyl methacrylate are less likely to form a swelled diffuse layer, the hydrophilic polymer is preferably a copolymer of these units and units such as vinyl pyrrolidone, ethylene glycol, or vinyl alcohol. From the viewpoint of the balance between the water solubility and the hydrophobicity, the hydrophilic polymer is more preferably a copolymer of vinyl pyrrolidone and vinyl acetate, copolymer of vinyl pyrrolidone and methyl methacrylate, copolymer of ethylene glycol and vinyl acetate, or copolymer of ethylene glycol and methyl methacrylate.

Hydrophilic polymers suitable for formation of the diffuse layer are preferably those having favorable balances between the water solubility and the hydrophobicity in a single molecule. The hydrophilic polymer is preferably a random copolymer or an alternating copolymer. In cases where the copolymer has an ester group, the molar ratio of ester group units is preferably 0.3 to 0.7.

The abundance ratio of carbon atoms derived from ester groups to the total carbon atoms in the portion from the surface of the hollow fiber membrane in the inlet-side space to a depth of 10 nm can be calculated by carrying out measurement by ESCA at a measurement angle of 90°, and splitting the peak of the component derived from ester groups from the entire C1s peak in the portion from the hollow fiber membrane surface to a depth of about 10 nm. More specifically, the peak of the component derived from ester groups is split from the entire peak of the following five components constituting C1s: the component mainly derived from CHx, C-C, C=C, and C-S; the component mainly derived from C-O and C-N; the component derived from $\pi$-$\pi^*$ satellite; the component derived from C=O; and the component derived from ester groups. By calculating the peak area ratio of the component derived from ester groups to the area of the entire C1s peak (hereinafter referred to as "ester group-derived peak area ratio"), the abundance ratio of carbon atoms derived from ester groups to the total carbon atoms can be calculated. The peak of the component derived from ester groups appears at + 4.0 to 4.2 eV from the main peak of the component derived from CHx and the

like (near 285 eV). The value obtained by multiplying the carbon amount of C1s (atomic percent) by the ester group-derived peak area ratio (the measurement is carried out at three positions, and the mean of the measured values (rounded to the ones place) is calculated; in cases where the ester group-derived peak area ratio is not more than 0.4%, the ratio is regarded as below the detection limit) is the abundance ratio of carbon atoms derived from ester groups to the total carbon atoms on the hollow fiber membrane surface in the inlet-side space. The abundance ratio of carbon atoms derived from ester groups to the total carbon atoms is preferably not less than 0.1 atomic percent, more preferably not less than 0.5 atomic percent. The abundance ratio of carbon atoms derived from ester groups is preferably not more than 10 atomic percent, more preferably not less than 5 atomic percent, still more preferably not less than 1 atomic percent.

For retaining the hydrophilic polymer on the hollow fiber membrane surface, it is advantageous for the hydrophilic polymer to have a large number of crosslinking points, that is, to have a high weight average molecular weight. However, in cases where the weight average molecular weight is too high, it is difficult to keep the membrane surface in a uniform state on the hollow fiber membrane surface because of its high viscosity and gelation, so that a swelled diffuse layer cannot be formed. On the other hand, in cases where the weight average molecular weight is too low, elution of the hydrophilic polymer may occur. Thus, the weight average molecular weight of the hydrophilic polymer is preferably 5000 to 1,500,000, more preferably 10,000 to 1,000,000.

The hydrophilic polymer may have a single weight average molecular weight, or may be a mixture of a plurality of kinds of hydrophilic polymers having different weight average molecular weights. The hydrophilic polymer may be prepared by purifying a commercial product such that it has a narrowed weight average molecular weight distribution.

In cases where polyvinyl pyrrolidone (hereinafter described as PVP) is used as the hydrophilic polymer, those referred to as K15 to K120 are preferred. From the viewpoint of increasing the hydrophilicity, the weight average molecular weight of the PVP is preferably not less than 10,000, more preferably not less than 40,000. PVP is a water-soluble polymer produced by vinyl polymerization of N-vinyl pyrrolidone, and products having various molecular weights are commercially available under the trade names of, for example, Luvitec (registered trademark), which is manufactured by BASF; Plasdone (registered trademark), which is manufactured by ISP; and Pitzcol (registered trademark), which is manufactured by DKS Co. Ltd.

Commercially available copolymers of PVP and vinyl acetate have weight ratios of PVP:vinyl acetate of (7:3), (6:4), (5:5), (3:7), and the like. It is preferred to use, for example, VA64 which has a weight ratio of 6/4, VA73, VA55, VA37, or PVC55 of Kollidon (registered trademark), manufactured by BASF.

The method for controlling the abundance ratio of hydrophilic polymers on the hollow fiber membrane surface in the inlet-side space is not limited, and examples of the method include a method in which the hydrophilic polymer is mixed with the membrane-forming liquid in the production process of the hollow fiber membrane, a method in which a hydrophilic-polymer solution is brought into contact with the surface during the membrane formation, and a method in which the surface is coated with the hydrophilic polymer. In these methods, after giving the hydrophilic polymer to the surface, the hydrophilic polymer may be cross-linked to the hollow fiber membrane by, for example, radiation or thermal treatment. By this, elution of the hydrophilic polymer from the hollow fiber membrane surface can be suppressed. Alternatively, the hydrophilic polymer may be immobilized on the hollow fiber membrane by chemical reaction.

In the thermal cross-linking, in which the obtained hollow fiber membrane is heated, hydrophilic polymers present on the hollow fiber membrane surface are cross-linked to each other. From the viewpoint of allowing the cross-linking between the hydrophilic polymers while preventing degradation reaction, the temperature during the thermal cross-linking is preferably 120 to 250°C, more preferably 130 to 200°C. The length of time of the thermal cross-linking is preferably 1 to 10 hours, more preferably 3 to 8 hours.

In the radiation cross-linking, in which the obtained hollow fiber membrane is irradiated with radiation, the hydrophilic polymer is cross-linked to the polysulfone-based polymer. From the viewpoint of allowing the cross-linking reaction to proceed while preventing degradation reaction, the radiation dose during the radiation cross-linking is preferably 5 to 75 kGy, more preferably 10 to 50 kGy. As the radiation for the irradiation, $\alpha$-ray, $\beta$-ray, X-ray, $\gamma$-ray, or electron beam is employed. Among these, $\gamma$-ray and electron beam is preferred. For allowing the cross-linking reaction to proceed more easily, water is preferably added to the hollow fiber membrane to be subjected to the radiation cross-linking.

In the washed platelets, platelets are suspended in a preservation solution having high storage stability for the platelet function, instead of the blood plasma, which is removed during the production. In the recovering step, a preservation solution containing bicarbonate is preferably used as the preservation solution for recovery of platelets in the hollow fiber membrane module. In the filtration step or the washing step, a preservation solution containing bicarbonate is preferably used since it has high affinity with platelets.

In cases where a preservation solution containing bicarbonate is allowed to flow into the hollow fiber membrane module, there is a problem of inclusion of bubbles into the hollow fiber membrane module, since carbon dioxide gas is generated from bicarbonate. If bubbles are included in the hollow fiber membrane module, platelet aggregation due to contact of platelets with the bubbles is likely to occur, leading to a low platelet recovery rate. Moreover, the bubbles block the channel in the inlet-side space of the hollow fiber membrane module, and prevent the liquid flow. As a result, the washing and the recovery become difficult, leading to decreases in the protein removal rate and the platelet recovery

rate. By connecting a circuit having an air chamber upstream of the platelet suspension inlet, introduction of bubbles generated from the preservation solution into the hollow fiber membrane module can be suppressed. For sufficient removal of bubbles generated from the preservation solution, the capacity of the air chamber is preferably not less than 1 mL. On the other hand, in cases where the capacity of the air chamber is small, retention of platelets in the chamber is suppressed, and the platelet recovery rate increases as a result. Thus, the capacity of the air chamber is preferably not more than 30 mL.

For carrying out dead-end filtration of a platelet suspension using a hollow fiber membrane module, the following methods can be employed: constant pressure filtration, in which the platelet suspension is fed at a constant pressure; and constant rate filtration, in which the platelet suspension is fed at a constant rate. Since, as mentioned above, the linear velocity of the flow of the platelet suspension influences the platelet recovery rate, the dead-end filtration is preferably carried out by constant rate filtration, in which the flow rate can be controlled. Examples of means for feeding the platelet suspension at a constant rate include syringe pumps and roller pumps. Roller pumps are preferred since they can feed a large amount of the platelet suspension. On the other hand, since squeezing by a roller pump is likely to cause generation of bubbles from the preservation solution containing bicarbonate, it is preferred to arrange a roller pump, air chamber, and hollow fiber membrane module in this order from the upstream side where the platelet suspension is fed.

In the present invention, a hollow fiber membrane module for the internal pressure method is especially preferably used since, in such a case, unevenness of the feed rate of the platelet suspension is less likely to occur; the hollow fiber membranes can be uniformly used; and retention of platelets can be suppressed.

## EXAMPLES

The present invention is described below in detail by way of Examples. However, the present invention is not limited thereto.

Measurement of Water Permeability:

The water permeability of the hollow fiber membrane module is calculated by cutting hollow fiber membranes out from the hollow fiber membrane module, and measuring the water permeability per unit membrane area of the hollow fiber membranes, followed by multiplying the measured value by the membrane area of the hollow fiber membranes contained in the hollow fiber membrane module. First, the water permeability per unit membrane area can be measured by the following method. Hollow fiber membranes contained in the hollow fiber membrane module were cut out. The hollow fiber membranes were inserted into a plastic pipe, and both ends of the hollow fiber membranes were potted to the inner walls at both ends of the plastic pipe, to prepare a mini-module having an effective length of 10 cm. The number of the hollow fiber membranes was adjusted such that the membrane area of the mini-module was 0.003 $m^2$. In cases where a hollow fiber membrane module for the internal pressure method is used, the membrane area corresponds to the membrane area based on the inner diameter. In cases where a hollow fiber membrane module for the external pressure method is used, the membrane area corresponds to the membrane area based on the outer diameter. The membrane area of the mini-module was calculated according to the following Equation 5. Here, when the hollow fiber membrane module contained two or more kinds of hollow fiber membranes, the respective kinds of hollow fiber membranes were used such that the ratios of their numbers were the same between the hollow fiber membrane module and the mini-module, and, in the calculation of the membrane area, the values calculated for the respective kinds of hollow fiber membranes according to Equation 5 were integrated.

$$A_{mini} = D \times \pi \times L \times n \text{ ... Equation 5}$$

$A_{mini}$: Membrane area of the mini-module ($m^2$)
D: Hollow fiber diameter (m) (inner diameter in the internal pressure method, or outer diameter in the external pressure method)
$\pi$: Circumference ratio
L: Effective length (m)
n: Number of hollow fiber membranes

To the mini-module prepared, a water pressure of $1.3 \times 10^4$ Pa was applied, and the amount of water released per unit time into the side where the filtrate from the hollow fiber membranes is obtained was measured. In terms of the direction of application of the water pressure, when the hollow fiber membrane module was to be used by the internal

pressure method, the water pressure was applied to the mini-module by the internal pressure method. When the hollow fiber membrane module was to be used by the external pressure method, the water pressure was applied to the mini-module by the external pressure method. According to the following Equation 6, the water permeability of the hollow fiber membranes was calculated.

$$F_M = Q / (T \times P \times A_{mini}) \text{ ... Equation 6}$$

$F_M$: Water permeability of the hollow fiber membranes ($mL/hr/Pa/m^2$)
Q: Amount of water released (mL)
T: Length of time of application of the water pressure (hr)
P: Water pressure (Pa)
$A_{mini}$: Membrane area of the mini-module ($m^2$)

Subsequently, the membrane area of the hollow fiber membrane module was calculated according to Equation 7. Here, when the hollow fiber membrane module contained two or more kinds of hollow fiber membranes, the values calculated for the respective kinds of hollow fiber membranes according to Equation 7 were integrated. The water permeability of the hollow fiber membrane module was calculated according to the following Equation 8.

$$A_{MD} = D \times \pi \times L \times n \text{ ... Equation 7}$$

$A_{MD}$: Membrane area of the hollow fiber membrane module ($m^2$)
D: Hollow fiber diameter (m) (inner diameter in the internal pressure method, or outer diameter in the external pressure method)
$\pi$: Circumference ratio
L: Effective length (m)
n: Number of hollow fiber membranes

$$F_{MD} = F_M \times A_{MD} \text{ ... Equation 8}$$

$F_{MD}$: Water permeability of the hollow fiber membrane module (mL/Pa/hr)
$F_M$: Water permeability of the hollow fiber membranes ($mL/hr/Pa/m^2$)
$A_{MD}$: Membrane area of the hollow fiber membrane module ($m^2$)

Measurement of Filtration Pressure of Platelet Suspension:

By measuring the concentration of the platelet suspension, a platelet suspension at a concentration of $1.25 \times 10^9$ platelets/mL was prepared. When the platelet concentration was low, platelets were precipitated by centrifugation, and the resulting supernatant was removed to concentrate the platelets. When the platelet concentration was high, a part of the platelet suspension was taken, and subjected to centrifugation to precipitate platelets, followed by adding the resulting supernatant to the original platelet suspension, thereby diluting the platelet suspension. The washed platelet outlet was closed, and the platelet suspension inlet and the filtrate outlet were opened. To perform dead-end filtration, 200 mL of the platelet suspension whose concentration was adjusted was allowed to flow into the platelet suspension inlet at a rate of 50 mL/min. The pressure at the platelet suspension inlet P1, the pressure at the washed platelet outlet P2, and the pressure at the filtrate outlet Po were measured. According to the following Equation 9, the filtration pressure was calculated.

$$\text{Filtration pressure} = (P1 + P2) / 2 - Po \text{ ... Equation 9}$$

Measurement of Ratio of Pore Areas on Membrane Surface:

Using a scanning electron microscope, an image of the membrane surface of the hollow fiber membrane in the inlet-side space, which is the side contacting the platelet suspension, was taken at a magnification of x1000. Subsequently,

image processing was carried out using Matrox Inspector 2.2 (Matrox Electronic Systems Ltd.) such that the pore areas were painted white, while the remaining area was painted black. The number of the pores shown in white (hereinafter referred to as "total opening number") and the total pixel number of the pores shown in white (hereinafter referred to as "total opening area") were determined, and the ratio of pore areas and the average pore size were calculated for each image according to the following Equation 10 and Equation 11. These measurement operations were carried out for 10 random positions on each of five hollow fiber membranes, that is, a total of 50 times, and the mean for the total of 50 images was calculated as the ratio of pore areas on the hollow fiber membrane surface in the inlet-side space. The above-described images were taken at a magnification of x1000 under the following conditions.

Image size: $655 \times 740$ pixels

Image resolution: 0.140845 μm/pixel

Image area S: 9615.2 $\mu m^2$ (92.3 μm length × 104.2 μm width)

$$\text{Ratio of pore areas (\%)} = \text{total opening area} / \text{image size} \times 100 \quad \ldots \text{Equation 10}$$

$$\text{Average pore size } (\mu m) = \text{total opening number} \times (\text{total opening area} / \pi)^{0.5} \quad \ldots \text{Equation 11}$$

Measurement of Abundance Ratio of Hydrophilic Polymers to Total Molecules on Hollow Fiber Membrane Surface:

The surface of the hollow fiber membrane in the inlet-side space is exposed, and the membrane is then rinsed with ultrapure water, followed by drying at room temperature at 0.5 Torr for 10 hours provide a measurement sample. The sample is set in an X-ray photoelectron spectrometer (which may be, for example, ESCALAB 220i-XL, manufactured by Thermo Fisher Scientific Inc.), and the angle of the detector with respect to the angle of incidence of X-ray is adjusted such that the measurement angle becomes 90°, followed by performing the measurement. From the integrated intensity of the spectrum of each of Cls, N1s, and S2p, and the relative sensitivity coefficient specific to the apparatus, the abundance ratios of carbon atoms, nitrogen atoms, and sulfur atoms in the portion from the surface of the hollow fiber membrane in the inlet-side space to a depth of 10 nm are determined.

Here, for example, in cases where a polysulfone-based polymer and polyvinyl pyrrolidone are used as the membrane material, the measurement is carried out by XPS at a measurement angle of 90° to investigate the abundance ratios of carbon atoms, nitrogen atoms, and sulfur atoms in the portion from the surface of the membrane to a depth of 10 nm. According to the following Equation 12, the abundance ratio of hydrophilic polymers to the total molecules in the portion from the surface of the hollow fiber membrane in the inlet-side space to a depth of 10 nm can be calculated.

$$\text{Abundance ratio of hydrophilic polymers to the total molecules (\% by mass)} = N \times 111 / (N \times 111 + S \times 442) \quad \ldots \text{Equation 12}$$

N: Abundance ratio of nitrogen atoms
S: Abundance ratio of sulfur atoms
111: Number of repeat units of polyvinyl pyrrolidone
442: Number of repeat units of polysulfone-based polymer

Measurement of Abundance Ratio of Carbon Atoms Derived from Ester Groups to Total Carbon Atoms on Hollow Fiber Membrane Surface:

Similarly to the measurement of the abundance ratio of hydrophilic polymers on the hollow fiber membrane surface, measurement was carried out by ESCA at a measurement angle of 90°. The peak of the component derived from ester groups was split from the entire C1s peak obtained for the portion from the surface of the hollow fiber membrane in the inlet-side space to a depth of about 10 nm, and the abundance ratio of carbon atoms derived from ester groups

to the total carbon atoms was calculated.

More specifically, the peak of the component derived from ester groups is split from the entire peak of the following five components constituting C1s: the component mainly derived from CHx, C-C, C=C, and C-S; the component mainly derived from C-O and C-N; the component derived from $\pi$-$\pi$* satellite; the component derived from C=O; and the component derived from ester groups. By calculating the peak area ratio of the component derived from ester groups to the area of the entire C1s peak (hereinafter referred to as "ester group-derived peak area ratio"), the abundance ratio of carbon atoms derived from ester groups to the total carbon atoms can be calculated. The peak of the component derived from ester groups appears at + 4.0 to 4.2 eV from the main peak of the component derived from CHx and the like (near 285 eV). The value obtained by multiplying the carbon measurement is carried out at three positions, and the mean of the measured values (rounded to the ones place) is calculated; in cases where the ester group-derived peak area ratio is not more than 0.4%, the ratio is regarded as below the detection limit) is the abundance ratio of carbon atoms derived from ester groups to the total carbon atoms in the portion from the hollow fiber membrane surface in the inlet-side space to a depth of about 10 nm.

Protein Concentration Measurement:

The protein concentration measurement was carried out by the BCA method using a BCA PROTEIN ASSAY KIT (manufactured by THERMO SCIENTIFIC). A platelet suspension or washed platelets was/were centrifuged at 2000xg for 10 minutes, and the resulting supernatant was used as a measurement sample. First, in the measurement, BCA reagent and calibration curve samples were prepared. For dilution of each sample, M-sol, which is the preservation solution used for the production of the washed platelets, was used. According to the specification of the kit, BCA reagent was added to the calibration curve samples and the measurement sample. Each resulting mixture was stirred at room temperature for 10 seconds using a micromixer. Thereafter, the mixture was incubated at 37°C for 30 minutes. The sample was then allowed to cool to room temperature, and subjected to measurement of the absorbance at a wavelength of 562 nm. The wavelength for the measurement of the absorbance does not need to be strictly the same as long as it is within the range of about $\pm$20 nm from this wavelength. Using the calibration curve samples, a calibration curve for the protein concentration and the absorbance was prepared. By substituting the absorbance of the measurement sample into the formula of the calibration curve, the protein concentration of the measurement sample was determined.

Preparation of Washed Platelets:

Ten units of a platelet suspension contains $2\times10^{11}$ to $3\times10^{11}$ platelets, and its volume is about 200 mL. The number of platelets in the platelet suspension was measured using a multi-parameter automated hematology analyzer XT-1800i (manufactured by Sysmex Corporation). The protein concentration in the platelet suspension was measured.

By adding 52.2 mL of Meylon (registered trademark) manufactured by Otsuka Pharmaceutical Co., Ltd., 126.8 mL of ACD-A solution manufactured by Terumo Corporation, 3.2 mL of Magnesium Sulfate Corrective Injection (1 mEq/mL) manufactured by Otsuka Pharmaceutical Co., Ltd., and 71.6 mL of distilled water manufactured by Otsuka Pharmaceutical Co., Ltd., to 746.2 mL of Solacet F (registered trademark) manufactured by Terumo Corporation, and mixing the resulting mixture, 1 L of M-sol was prepared as an artificial preservation solution.

Two hundred milliliters of 10 units of a platelet suspension was diluted with 2 volumes, that is, 400 mL, of M-sol. A circuit tube was filled with M-sol, and connected to the platelet suspension inlet of the hollow fiber membrane module. In the circuit, an air chamber having a capacity of 13 mL was arranged between the roller pump and the hollow fiber membrane module. M-sol was allowed to flow through the hollow fiber membrane module to replace the liquid in the hollow fiber membrane module with M-sol. The washed platelet outlet was closed, and the platelet suspension inlet and the filtrate outlet were opened. The diluted platelet suspension was fed from the platelet suspension inlet of the hollow fiber membrane module at a flow rate of 50 mL/min. The platelet suspension was allowed to flow through the inlet-side space of the hollow fiber membrane module, and filtered through the hollow fiber membranes. The resulting filtrate was then allowed to flow through the filtrate-side space of the hollow fiber membrane module, and released from the filtrate outlet. In this process, platelets do not pass the hollow fiber membranes, and stay in the inlet-side space of the hollow fiber membrane module. Proteins and water, which pass the hollow fiber membranes, are discharged as a filtrate. After feeding the whole platelet suspension, 1000 mL of M-sol as a washing liquid was allowed to flow through the same channel at 50 mL/min. Subsequently, the filtrate outlet was closed, and the platelet suspension inlet and the washed platelet outlet were opened. As a preservation solution, 200 mL of M-sol was fed from the platelet suspension inlet into the inlet-side space of the hollow fiber membrane module at a flow rate of 250 mL/min., and released from the washed platelet outlet. The platelet concentration and the protein concentration of the washed platelets obtained were measured. From the concentrations and the volumes of the platelet suspension and the washed platelets, the protein removal rate was calculated according to Equation 13, and the platelet recovery rate was calculated according to Equation 14.

$$\text{Protein removal rate (\%)} = (1 - (Co1 \times Vo) / (Ci1 \times Vi)) \times 100 \text{ ... Equation 13}$$

Co1: Protein concentration in the washed platelets (mg/mL)
Ci1: Protein concentration in the platelet suspension (mg/mL)
Vo: Volume of the washed platelets (mL)
Vi: Volume of the platelet suspension (mL)

$$\text{Platelet recovery rate (\%)} = ((Co2 \times Vo) / (Ci2 \times Vi)) \times 100 \text{ ... Equation 14}$$

Co2: Platelet concentration in the washed platelets (platelets/mL)
Ci2: Platelet concentration in the platelet suspension (platelets /mL)
Vo: Volume of the washed platelets (mL)
Vi: Volume of the platelet suspension (mL)

(Example 1)

A mixture composed of 15 parts of Udel (registered trademark) polysulfone (P3500, Solvay), 8 parts of PVP (K90, ISP), 75 parts of DMAC, and 2 parts of water was mixed at 90°C, and the resulting solution was incubated at 50°C, to prepare a membrane-forming liquid. To a mixed solution composed of 80 parts of DMAC and 20 parts of water, 30 parts of PVP (K30, ISP) was added, and the resulting mixture was mixed to prepare a solution as a core liquid.

Using an orifice-type double annular nozzle having an outer diameter of 1.0 mm and inner diameter of 0.7 mm, the membrane-forming liquid and the core liquid were discharged at the same time from the outer cylinder and the inner cylinder, respectively, and allowed to pass through a dry section at 30°C having a length of 70 mm, followed by immersion in a coagulation bath at 90°C containing a mixed solution of 85 parts of water and 15 parts of DMAC, thereby allowing coagulation. The resulting product was washed in warm water in a warm water bath at 80°C, and then wound into a reel, to obtain a hollow fiber membrane in the wet state. As a result of formation of the membrane at a membrane formation rate of 40 m/min, the inner diameter of the hollow fiber membrane became 300 μm and the membrane thickness of the hollow fiber membrane became 80 μm.

The obtained hollow fiber membrane in the wet state was cut into pieces each having a length of 0.4 m, and subjected to washing in warm water by immersion in a warm water bath at 90°C for 50 minutes. Subsequently, drying treatment was carried out at 100°C for 10 hours, and thermal cross-linking treatment was then carried out in a heat dryer at 170°C for 5 hours, to obtain hollow fiber membranes.

From the hollow fiber membranes obtained, a hollow fiber membrane module was prepared as follows. First, a bundle of 6864 hollow fiber membranes obtained by the membrane formation operation described above was inserted into a cylindrical plastic member having an inner diameter of 50 mm and a length of 290 mm in which a filtrate outlet is provided at a position 21 mm distant from an end face of the cylindrical member, that is, at a position 7% distant from an end face of the cylindrical member with respect to the end face-end face distance. The ends were sealed with a potting material composed of a polyurethane resin to provide partition walls, and the potting material was cut along the direction parallel to the cross-section of the cylindrical member such that the hollow fiber membranes at both end faces open toward the outside. At the end of the cylindrical member in the side more distant from the filtrate outlet, a header with a capacity of 8.2 mL having a platelet suspension inlet was attached, and, at the other end, a header with a capacity of 8.2 mL having a washed platelet outlet was attached. Into the housing containing the hollow fiber membranes, an aqueous solution of 1000 ppm VA64 in which ethanol is dissolved at 0.1% by mass was filled, and the hollow fiber membranes were irradiated with 25 kGy of γ-ray from the outside of the housing to perform radiation irradiation cross-linking, thereby obtaining a hollow fiber membrane module. The resulting hollow fiber membrane module is for use in the internal pressure method, and the inlet-side space of the hollow fiber membrane module corresponds to the insides of both headers and the hollow portions of the hollow fiber membranes.

The effective length (L) of the hollow fiber membrane was 255 mm, and the cross-sectional area (A) of the hollow fiber membrane hollow portions (cross-sectional area of the inlet-side space vertical to the longitudinal direction of the housing) was 0.00049 $m^2$. Thus, the ratio (L/A) of the effective length (L) of the hollow fiber membrane to the cross-sectional area (A) of the hollow fiber membrane hollow portions was 520 $m^{-1}$. The capacity of the inlet-side space of the hollow fiber membrane module was 155 mL. The water permeability of the hollow fiber membrane module was 125 mL/Pa/hr. The ratio of pore areas of the hollow fiber membrane surface in the inlet-side space was 17.3%; the abundance ratio of hydrophilic polymers in the portion from the surface of the hollow fiber membrane in the inlet-side space to a depth of 10 nm was 54.2%; and the peak area percentage of carbon atoms derived from ester groups with

respect to the total carbon atoms in the portion from the surface of the hollow fiber membrane in the inlet-side space to a depth of 10 nm was 0.5 atomic percent. In measurement of the filtration pressure, the maximum pressure was 5 kPa.

When washed platelets were prepared, the platelet recovery rate was 97.5%, and the protein removal rate was 93.5%. Since the water permeability of the hollow fiber membrane module was high, the filtration pressure was less likely to increase. Therefore, the platelet recovery rate was high in dead-end filtration. Washed platelets with a high platelet concentration and a low protein concentration could be produced. The above results are shown as Example 1 in Table 1.

(Example 2)

A hollow fiber membrane module was prepared in the same manner as in Example 1 except that the inner diameter of the cylindrical member was 44 mm; the header capacity was 6.4 mL; and the number of hollow fiber membranes inserted was 5243.

The effective length (L) of the hollow fiber membrane was 255 mm, and the cross-sectional area (A) of the hollow fiber membrane hollow portions (cross-sectional area of the inlet-side space vertical to the longitudinal direction of the housing) was 0.00037 $m^2$. Thus, the ratio (L/A) of the effective length (L) of the hollow fiber membrane to the cross-sectional area (A) of the hollow fiber membrane hollow portions was 689 $m^{-1}$. The capacity of the inlet-side space of the hollow fiber membrane module was 118 mL. The water permeability of the hollow fiber membrane module was 95 mL/Pa/hr. The ratio of pore areas of the hollow fiber membrane surface in the inlet-side space was 17.3%; the abundance ratio of hydrophilic polymers in the portion from the surface of the hollow fiber membrane in the inlet-side space to a depth of 10 nm was 54.2%; and the peak area percentage of carbon atoms derived from ester groups with respect to the total carbon atoms in the portion from the surface of the hollow fiber membrane in the inlet-side space to a depth of 10 nm was 0.5 atomic percent. In measurement of the filtration pressure, the maximum pressure was 7 kPa.

When washed platelets were prepared, the platelet recovery rate was 96.5%, and the protein removal rate was 98.6%. The above results are shown as Example 2 in Table 1.

(Example 3)

A hollow fiber membrane module was prepared in the same manner as in Example 1 except that the inner diameter of the cylindrical member was 40 mm; the header capacity was 5.3 mL; and the number of hollow fiber membranes inserted was 4494.

The effective length (L) of the hollow fiber membrane was 255 mm, and the cross-sectional area (A) of the hollow fiber membrane hollow portions (cross-sectional area of the inlet-side space vertical to the longitudinal direction of the housing) was 0.00032 $m^2$. Thus, the ratio (L/A) of the effective length (L) of the hollow fiber membrane to the cross-sectional area (A) of the hollow fiber membrane hollow portions was 796 $m^{-1}$. The capacity of the inlet-side space of the hollow fiber membrane module was 101 mL. The water permeability of the hollow fiber membrane module was 82 mL/Pa/hr. The ratio of pore areas of the hollow fiber membrane surface in the inlet-side space was 17.3%; the abundance ratio of hydrophilic polymers in the portion from the surface of the hollow fiber membrane in the inlet-side space to a depth of 10 nm was 54.2%; and the peak area percentage of carbon atoms derived from ester groups with respect to the total carbon atoms in the portion from the surface of the hollow fiber membrane in the inlet-side space to a depth of 10 nm was 0.5 atomic percent. In measurement of the filtration pressure, the maximum pressure was 7 kPa.

When washed platelets were prepared, the platelet recovery rate was 82.9%, and the protein removal rate was 98.1%. The above results are shown as Example 3 in Table 1.

(Example 4)

A hollow fiber membrane module was prepared in the same manner as in Example 1 except that the inner diameter of the cylindrical member was 38 mm; the header capacity was 4.8 mL; and the number of hollow fiber membranes inserted was 3995.

The effective length (L) of the hollow fiber membrane was 255 mm, and the cross-sectional area (A) of the hollow fiber membrane hollow portions (cross-sectional area of the inlet-side space vertical to the longitudinal direction of the housing) was 0.00028 $m^2$. Thus, the ratio (L/A) of the effective length (L) of the hollow fiber membrane to the cross-sectional area (A) of the hollow fiber membrane hollow portions was 910 $m^{-1}$. The capacity of the inlet-side space of the hollow fiber membrane module was 90 mL. The water permeability of the hollow fiber membrane module was 72 mL/Pa/hr. The ratio of pore areas of the hollow fiber membrane surface in the inlet-side space was 17.3%; the abundance ratio of hydrophilic polymers in the portion from the surface of the hollow fiber membrane in the inlet-side space to a depth of 10 nm was 54.2%; and the peak area percentage of carbon atoms derived from ester groups with respect to the total carbon atoms in the portion from the surface of the hollow fiber membrane in the inlet-side space to a depth of

10 nm was 0.5 atomic percent. In measurement of the filtration pressure, the maximum pressure was 20 kPa.

When washed platelets were prepared, the platelet recovery rate was 69.9%, and the protein removal rate was 97.0%. The above results are shown in Table 1.

(Example 5)

A hollow fiber membrane module was prepared in the same manner as in Example 1 except that the length of the cylindrical member was 220 mm, and the number of hollow fiber membranes inserted was 4600.

The effective length (L) of the hollow fiber membrane was 198 mm, and the cross-sectional area (A) of the hollow fiber membrane hollow portions (cross-sectional area of the inlet-side space vertical to the longitudinal direction of the housing) was 0.00032 $m^2$. Thus, the ratio (L/A) of the effective length (L) of the hollow fiber membrane to the cross-sectional area (A) of the hollow fiber membrane hollow portions was 614 $m^{-1}$. The capacity of the inlet-side space of the hollow fiber membrane module was 88 mL. The water permeability of the hollow fiber membrane module was 85 mL/Pa/hr. The ratio of pore areas of the hollow fiber membrane surface in the inlet-side space was 17.3%; the abundance ratio of hydrophilic polymers in the portion from the surface of the hollow fiber membrane in the inlet-side space to a depth of 10 nm was 54.2%; and the peak area percentage of carbon atoms derived from ester groups with respect to the total carbon atoms in the portion from the surface of the hollow fiber membrane in the inlet-side space to a depth of 10 nm was 0.5 atomic percent. In measurement of the filtration pressure, the maximum pressure was 6 kPa.

When washed platelets were prepared, the platelet recovery rate was 96.7%, and the protein removal rate was 98.2%. The above results are shown in Table 1.

(Comparative Example 1)

A hollow fiber membrane module was prepared in the same manner as in Example 1 except that the inner diameter of the cylindrical member was 19 mm; the header capacity was 1.2 mL; and the number of hollow fiber membranes inserted was 1000.

The effective length (L) of the hollow fiber membrane was 255 mm, and the cross-sectional area (A) of the hollow fiber membrane hollow portions (cross-sectional area of the inlet-side space vertical to the longitudinal direction of the housing) was 0.00007 $m^2$. Thus, the ratio (L/A) of the effective length (L) of the hollow fiber membrane to the cross-sectional area (A) of the hollow fiber membrane hollow portions was 3642 $m^{-1}$. The capacity of the inlet-side space of the hollow fiber membrane module was 23 mL. The water permeability of the hollow fiber membrane module was 19 mL/Pa/hr. The ratio of pore areas of the hollow fiber membrane surface in the inlet-side space was 17.3%; the abundance ratio of hydrophilic polymers in the portion from the surface of the hollow fiber membrane in the inlet-side space to a depth of 10 nm was 54.2%; and the peak area percentage of carbon atoms derived from ester groups with respect to the total carbon atoms in the portion from the surface of the hollow fiber membrane in the inlet-side space to a depth of 10 nm was 0.5 atomic percent. In measurement of the filtration pressure, the maximum pressure was 64 kPa.

When washed platelets were prepared, the platelet recovery rate was 32%, and the protein removal rate was 98.2%. Compared to Example 1, Comparative Example 1 had a lower water permeability of the hollow fiber membranes, higher L/A, and smaller capacity of the inlet-side space of the hollow fiber membrane module. Therefore, a pressure increase was likely to occur due to platelet aggregation during the dead-end filtration, resulting in a low platelet recovery rate. The above results are shown in Table 1.

[Table 1]

| | Water permeability (ml/Pa/hr) | Capacity of feed side space (ml) | L/A ($m^{-1}$) | Maximum pressure (kPa) | Platelet recovery rate (%) | Protein removal rate (%) |
|---|---|---|---|---|---|---|
| Example 1 | 125 | 155 | 520 | 5 | 97.5 | 93.5 |
| Example 2 | 95 | 118 | 689 | 7 | 96.5 | 98.6 |
| Example 3 | 82 | 101 | 796 | 7 | 82.9 | 98.1 |
| Example 4 | 72 | 90 | 910 | 20 | 69.9 | 97.0 |
| Example 5 | 85 | 88 | 614 | 6 | 96.7 | 98.2 |
| Comparative Example 1 | 19 | 23 | 3642 | 64 | 32.0 | 98.2 |

INDUSTRIAL APPLICABILITY

By using the hollow fiber membrane module of the present invention, impurities such as proteins can be efficiently removed from a platelet suspension without lowering the platelet concentration. Thus, washed platelets with a low protein concentration and a high platelet concentration can be produced.

DESCRIPTION OF SYMBOLS

1, Hollow fiber membrane module for the internal pressure method; 2, cylindrical member; 3, header; 4, header; 5, hollow fiber membrane; 6, platelet suspension inlet; 7, washed platelet outlet; 8, filtrate outlet; 9, partition wall; 10, partition wall; 11, inlet-side space; 12, filtrate-side space; 13, hollow fiber membrane hollow portion; 14, hollow fiber membrane module for the external pressure method; 15, air chamber; 16, roller pump; 17, tube clamp.

## Claims

**1.** A hollow fiber membrane module for washing platelets by removal of impurities from a platelet suspension, comprising:

a housing having a platelet suspension inlet, washed platelet outlet, and filtrate outlet; and
a hollow fiber membrane for filtering said platelet suspension, wherein pores through which said platelets do not pass, while said impurities pass, are formed, said hollow fiber membrane being arranged inside said housing;

wherein
the capacity of the inlet-side space which communicates with said platelet suspension inlet and stores said platelet suspension before being filtered through said hollow fiber membrane in said housing is 30 to 400 mL, and the module water permeability is 50 to 300 mL/Pa/hr.

**2.** The hollow fiber membrane module according to claim1, wherein the ratio (L/A) of the effective length (L) of said hollow fiber membrane to the cross-sectional area (A) of said inlet-side space vertical to the longitudinal direction of said housing is 250 to 1300 $m^{-1}$.

**3.** The hollow fiber membrane module according to claim 1 or 2, wherein the maximum pressure of filtration pressure during dead-end filtration of 200 mL of a platelet suspension containing $1.25\times10^9$ platelets/mL at a flow rate of 50 mL/min is not more than 30 kPa.

**4.** The hollow fiber membrane module according to any one of claims 1 to 3, wherein the ratio of pore areas on the surface of said hollow fiber membrane facing the inlet-side space is 10 to 30%.

**5.** The hollow fiber membrane module according to any one of claims 1 to 4, wherein said hollow fiber membrane is a membrane composed of a polysulfone-based polymer.

**6.** The hollow fiber membrane module according to any one of claims 1 to 5, wherein the abundance ratio of hydrophilic polymers to the total molecules from the surface of said hollow fiber membrane facing the inlet-side space to a depth of 10 nm is 40 to 60% by mass.

**7.** The hollow fiber membrane module according to any one of claims 1 to 6, wherein the abundance ratio of carbon atoms derived from ester groups to the total carbon atoms from the surface of said hollow fiber membrane facing the inlet-side space to a depth of 10 nm is 0.1 to 10 atomic percent.

**8.** A platelet suspension washing device comprising:

the hollow fiber membrane module according to any one of claims 1 to 7; and
an air chamber which is arranged upstream of said platelet suspension inlet, and has a capacity of 1 to 30 mL.

**9.** The platelet suspension washing device according to claim 8, wherein a roller pump is arranged upstream of said air chamber along the liquid current of said platelet suspension.

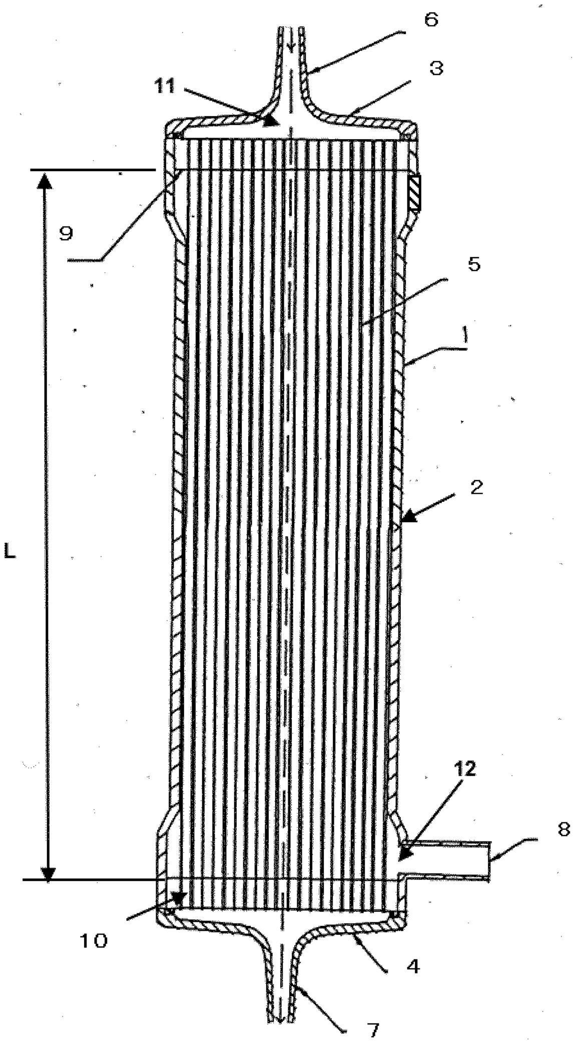


Fig. 1

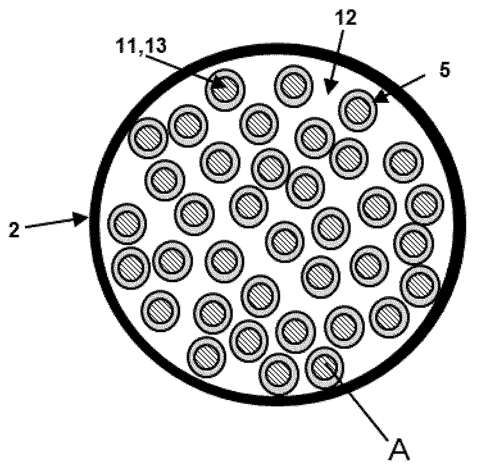


Fig. 2

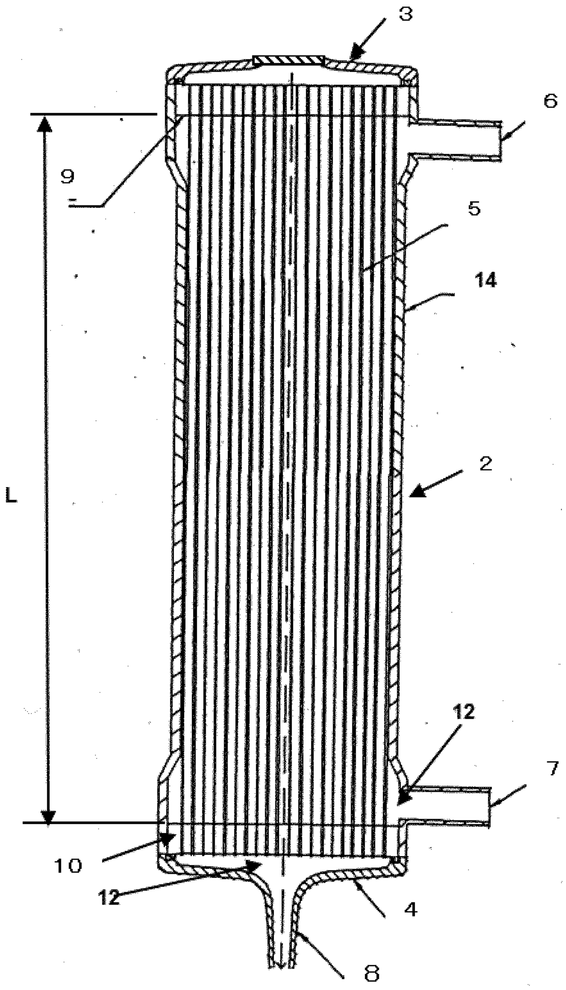


Fig. 3

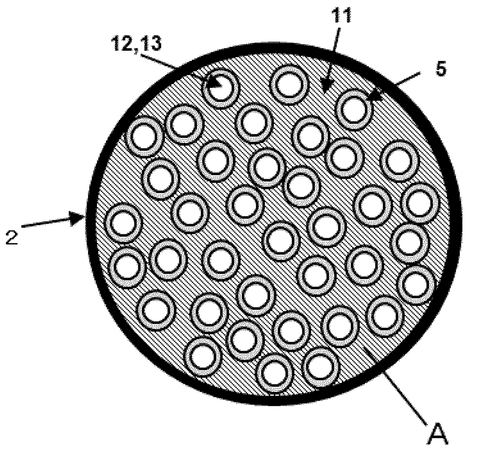


Fig. 4

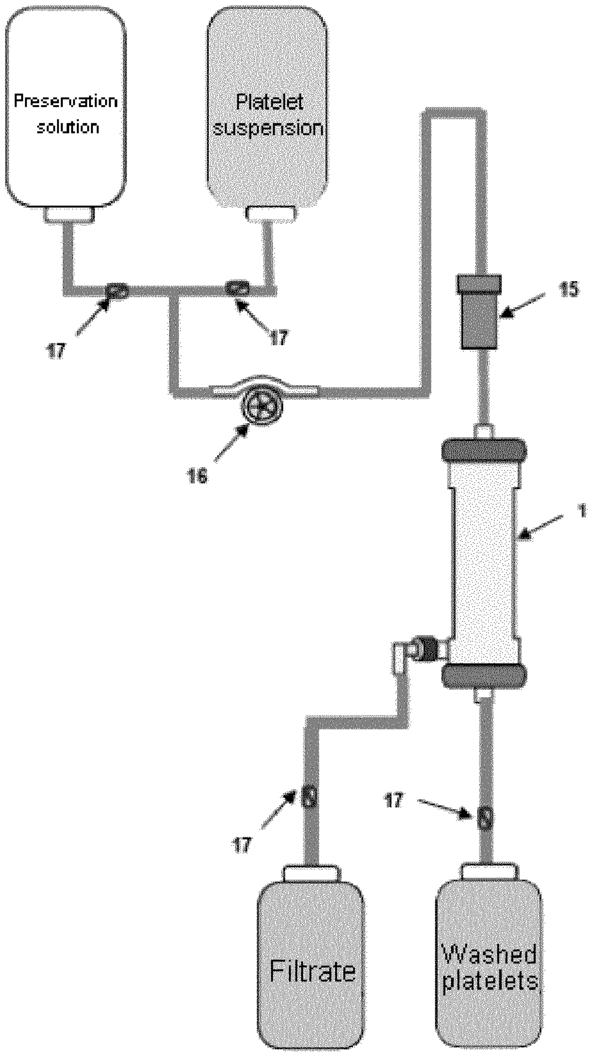
Preservation solution
Platelet suspension
15
17
17
16
1
17
17
17
Filtrate
Washed platelets

Fig. 5

**INTERNATIONAL SEARCH REPORT**

International application No. PCT/JP2015/054562

A. CLASSIFICATION OF SUBJECT MATTER
*A61M1/02*(2006.01)i, *B01D63/02*(2006.01)i, *B01D69/08*(2006.01)i, *B01D71/68* (2006.01)i

According to International Patent Classification (IPC) or to both national classification and IPC

B. FIELDS SEARCHED

Minimum documentation searched (classification system followed by classification symbols)
A61M1/02, B01D63/02, B01D69/08, B01D71/68

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 9-108338 A (Toray Industries, Inc.), 28 April 1997 (28.04.1997), paragraphs [0004], [0005]; fig. 1 to 7 (Family: none) | 1-9 |
| A | JP 2006-255261 A (Kuraray Medical Inc.), 28 September 2006 (28.09.2006), paragraphs [0003] to [0006]; fig. 1 to 4 (Family: none) | 1-9 |
| A | JP 3-47268 A (Terumo Corp.), 28 February 1991 (28.02.1991), page 16, upper right column, line 5 to lower left column, line 13; fig. 1, 2 (Family: none) | 1-9 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 March 2015 (04.03.15) | 17 March 2015 (17.03.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 1171566 A
- JP 2012143554 A
- JP 2012176081 A